(19) Europäisches Patentamt / European Patent Office / Office européen des brevets

(11) **EP 2 787 966 B1**

(12) **EUROPEAN PATENT SPECIFICATION**

(45) Date of publication and mention
of the grant of the patent:
**06.02.2019 Bulletin 2019/06**

(21) Application number: **12808595.8**

(22) Date of filing: **06.12.2012**

(51) Int Cl.:
*A61K 8/46* (2006.01)  *A61K 8/73* (2006.01)
*A61K 8/81* (2006.01)  *A61Q 19/10* (2006.01)
*A61Q 5/02* (2006.01)

(86) International application number:
**PCT/US2012/068227**

(87) International publication number:
**WO 2013/086178 (13.06.2013 Gazette 2013/24)**

(54) **PERSONAL CARE COMPOSITIONS**

KÖRPERPFLEGEZUSAMMENSETZUNGEN

COMPOSITION DE SOINS PERSONNELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.12.2011 US 201161568667 P
08.06.2012 US 201261657145 P**

(43) Date of publication of application:
**15.10.2014 Bulletin 2014/42**

(73) Proprietor: **The Procter & Gamble Company
Cincinnati, OH 45202 (US)**

(72) Inventors:
• **WEI, Karl, Shiqing**
**Cincinnati, Ohio 45241 (US)**
• **JI, Wei**
**Cincinnati, Ohio 45241 (US)**
• **ARREDONDO, Victor, Manuel**
**Cincinnati, Ohio 45241 (US)**

(74) Representative: **Kremer, Véronique Marie
Joséphine
Procter & Gamble Service GmbH
IP Department
Sulzbacher Straße 40-50
65824 Schwalbach am Taunus (DE)**

(56) References cited:
**EP-A1- 1 510 203**        **WO-A1-2008/039440**
**WO-A2-2011/156672**    **WO-A2-2013/142672**
**DE-A1-102008 035 172**   **US-A1- 2006 079 417**
**US-A1- 2011 117 225**

• **M.L.McMhon: "The use of nonionic associative
polymers for the thickening and emulsifying of
personal care products", , June 2011 (2011-06),
pages 1-66, Retrieved from the Internet:
URL:http://digitalcommons.calpoly.edu/cgi/
viewcontent.cgi?article=1587&context=these s
[retrieved on 2016-11-11]**

**Description**

TECHNICAL FIELD

The present disclosure generally relates to personal care compositions comprising an associative polymer, a non-associative polymer.

BACKGROUND

Cleansing the skin is an activity that has been done for millennia. Skin cleansing and methods therefore have involved the utilization of soaps, body washes, and other personal care compositions. Personal care compositions can be structured to suspend and stabilize dispersions of benefit agents while maintaining physical integrity of the personal care compositions, and there are many ways to provide such structure. The ability to provide structure can be an important property for such personal care compositions, but it is also important for personal care compositions to have the ability to rapidly become micellar upon dilution for cleansing the skin and depositing benefit agents. Having too much structure in a personal care composition can result in poor performance while not having enough structure in a personal care composition can cause instability. Further, achieving a balance between these two properties can be a difficult task. Document US 2006/0079417 discloses a multi-phase personal care composition comprising an anionic surfactant and polymeric phase structurants. As such, it is desirable to effectively improve the stability of personal care compositions.

SUMMARY

A personal care composition comprising: a) a structured cleansing phase comprising: i) from 5% to 20%, by weight of the personal care composition, of STnS where n is between 0.5 and 2.7; ii) an amphoteric surfactant, a zwitterionic surfactant, or a combination thereof; and iii) a structuring system comprising from 0.01% to 0.03%, by weight of the personal care composition, of an associative polymer and from 0.01% to 5%, by weight of the personal care composition, of a non-associative polymer; wherein the associative polymer comprises acrylates/C10-C30 alkyl acrylate cross-polymer and b) a benefit phase comprising from 0.1% to 50%, by weight of the personal care composition, of a benefit agent; wherein the structured cleansing phase and the benefit phase are in physical contact.

DETAILED DESCRIPTION

While the specification concludes with the claims particularly pointing and distinctly claiming the invention, it is believed that the present invention will be better understood from the following description.

The devices, apparatuses, methods, components, and/or compositions of the present invention can include, consist essentially of, or consist of, the components of the present invention as well as other ingredients described herein. As used herein, "consisting essentially of' means that the devices, apparatuses, methods, components, and/or compositions may include additional ingredients, but only if the additional ingredients do not materially alter the basic and novel characteristics of the claimed devices, apparatuses, methods, components, and/or compositions.

All percentages and ratios used herein are by weight of the total composition and all measurements made are at 25°C, unless otherwise designated.

All measurements used herein are in metric units unless otherwise specified.

I. Definitions

As used herein, the following terms shall have the meaning specified thereafter:

"Anhydrous" refers to those compositions, and components thereof, which are substantially free of water.

"Associative polymer" refers to a water-dispersible polymer comprising hydrophobic groups at an end or pendants to a hydrophilic backbone.

"Multiphase" refers to personal care compositions comprising at least two phases of which can be chemically distinct (e.g., a structured cleansing phase and a benefit phase). Such phases can be in direct physical contact with one another. A personal care composition can be a multiphase personal care composition where phases of the personal care composition can be blended or mixed to a significant degree, but still be physically distinct. In these situations, the physical distinctiveness is undetectable to the naked eye. The personal care composition can also be a multiphase personal care composition where phases of the personal care composition can be made to occupy separate and distinct physical spaces inside a package where the phases can be stored. In such an arrangement, the phases can be stored such that they are not in direct contact with one another (i.e., the phases are not separated by a barrier and the phases are not emulsified or mixed to any significant degree). The personal care composition can also be a multiphase personal care composition where the phases are in physical contact and are visually distinct. Visually distinct phases can take many forms (e.g., phases can appear as striped, marbled). The personal care composition can also include a combination of one or more of the above multiphase personal care compositions. In one such arrangement, one blended multiphase personal care composition can be stacked with another blended multiphase personal care composition to form a striped configuration. Additionally, blended multiphase personal care compositions distinguishable by color can be stacked as stripes wherein the blended multiphase personal care compositions can be otherwise similar in average composition.

"Non-associative polymer" refers to a water-dispersible polymer with a relatively uniform hydrophilic backbone lacking hydrophobic groups.

"Package" refers to any suitable container for a personal care composition including but not limited to a bottle, tottle, tube, jar, non-aerosol pump, and combinations thereof.

"Personal care composition" refers to compositions intended for topical application to skin or hair. Personal care compositions can be rinse-off formulations in which the product can be applied topically to the skin and/or hair and subsequently rinsed from the skin and/or hair with water. The product could also be wiped off using a substrate. In either case, it is believed at least a portion of the product is left behind (i.e., deposited) on the skin. The personal care compositions can also be used as shaving aids. The personal care compositions can be extrudable or dispensable from a package. The personal care compositions can exhibit a viscosity from about 1,500 cP to about 1,000,000 cP as measured by a viscosity method as described in the commonly owned patent application published on November 11, 2004 under U.S. Publication No. 2004/0223991 A1 entitled, "Multiphase Personal Care Compositions" filed on May 7, 2004 by Wei, et al. The personal care compositions can be in the form of, for example, a liquid, semi-liquid cream, lotion, or gel and are intended for topical application to the skin and/or hair. Examples of personal care compositions can include but are not limited to shampoo, conditioning shampoo, body wash, moisturizing body wash, shower gels, skin cleansers, cleansing milks, hair and body wash, in shower body moisturizer, pet shampoo, shaving preparations, and cleansing compositions used in conjunction with a disposable cleansing cloth.

"STnS" refers to sodium trideceth(n) sulfate, wherein n can define the average number of moles of ethoxylate per molecule.

"Stable" refers to a personal care composition having a viscosity change of about 30% or less from an initial viscosity value after being rapidly aged for 10 days at 50°C.

"Structured" refers to having a rheology that can confer stability on the personal care composition. A degree of structure can be determined by characteristics determined by the Zero Shear Viscosity Method described below. Accordingly, a structured cleansing phase of the personal care composition can be considered to be structured if the structured cleansing phase has a Zero Shear Viscosity of about 20 Pascal-seconds (Pa-s) or more, about 200 Pa-s or more, about 500 Pa-s or more, about 1,000 Pa-s or more, about 1,500 Pa-s or more, or about 2,000 Pa-s or more. Other methods for determining characteristics which can define a degree of structure are described in U.S. Patent Application Serial No. 13/157,665.

The phrase "substantially free of' as used herein, unless otherwise specified, means that the personal care composition comprises less than about 2%, less than about 1%, less than about 0.5%, or even less than about 0.1% of the stated ingredient. The term "free of', as used herein, means that the personal care composition comprises 0% of the stated ingredient that is the ingredient has not been added to the personal care composition. However, these ingredients may incidentally form as a by-product or a reaction product of the other components of the personal care composition.

"Surfactant component" refers to a total of all anionic, nonionic, amphoteric, zwitterionic, and cationic surfactants in a phase. When calculations are based on the surfactant component, water and electrolytes can be excluded from the calculations involving the surfactant component since surfactants as manufactured can be diluted and neutralized.

"Visually distinct" generally refers to a region of the multiphase personal care composition having one average composition, as distinct from another region having a different average composition, wherein the regions can be visible to the unaided naked eye. This would not preclude distinct regions from comprising two similar multiphase personal care compositions or phases where one multiphase personal care composition or phase can comprise certain pigments, dyes, particles, and various optional ingredients, hence providing a region of different average composition (e.g., different textures or different colors).

II. Personal Care Compositions

High concentrations of surfactant can be required to avoid instability in multiphase personal care compositions due to the need for the multiphase personal care composition to become micellar upon dilution which in turn allows for the release and deposition of the benefit agent(s). Additionally, personal care compositions containing high concentrations of surfactant can be harsh on the skin and/or hair when used and can also require larger amounts of benefit agents and/or additional components to improve stability. As a result, there is a continued interest in improving the stability of personal care compositions.

Structured surfactants can help provide stability to a personal care composition while still allowing the personal care composition to become micellar upon dilution. Additionally, the enhanced stability provided by the structured surfactant can allow for the amount of surfactant to be lowered while still maintaining good use properties. The ability to use less surfactant and maintain stability allows for a reduction in cost and a milder product.

However, reducing the amount of surfactant in personal care compositions may negatively impact the deposition of the benefit agent(s) due, in part, to a reduction in the transition to micellar upon dilution and the inefficiency of the benefit agent(s) to be released from the system. Furthermore, the aforementioned issue may be exacerbated when the benefit agent is known to be difficult to deposit or when the benefit agent is a poor occlusive agent. In either case, in order to achieve a comparable effect from the benefit agent(s), a greater amount of the benefit agent(s) will usually be required in the personal care composition to compensate for the effect caused in part by the reduction in the amount of surfactant. This issue may potentially be addressed with the identification of a class of benefit agents that can achieve the effect desired even in low surfactant systems.

Associative polymers can be used within a structured cleansing phase of a personal care composition to provide structure and establish a desirable rheology for delivering benefit agents. However, it is believed that using an associative polymer in the structured cleansing phase can result in slower dissolution kinetics as associative monomers with hydrophobic end groups can anchor the associative polymer into structured surfactant hydrophobic domains. Taken together, the benefits and detriments of utilizing an associative polymer with a structured surfactant should be weighed and balanced in the formulation.

Surprisingly, it has been found that providing a structuring system including an associative polymer and a nonassociative polymer in the structured cleansing phase can improve the stability of a personal care composition. For example, Table 1 demonstrates a decrease of about 75% to about 78% in the viscosity of the personal care composition after ten days at 50°C, in accordance with the T-Bar Viscosity Method as described herein, when an associative polymer but not a non-associative polymer is included in the personal care composition. In contrast, as shown in Table 2, a decrease of only about 14% to about 25% can be observed in the viscosity of the personal care composition after ten days at 50°C when a non-associative polymer is included in a personal care composition also containing an associative polymer.

It has also been found that a personal care composition including an associative polymer and a non-associative polymer can maintain such stability while exhibiting improved deposition. Additionally, the inclusion of a non-associative polymer can allow for a reduction in the amount of the associative polymer included in the personal care composition while maintaining and/or enhancing stability of the personal care composition. For example, Inventive Examples 1-3 in Table 2 provided herein illustrate that the amount of the associative polymer present in the personal care composition can be reduced and yet the stability of the personal care composition may still be enhanced. In contrast, as shown in Comparative Examples 1-3 shown in Table 1, decreasing the amount of the associative polymer in the absence of a non-associative polymer may not enhance the stability of the personal care composition.

Surprisingly, it has also been found that the use of sucrose polyesters as benefit agents in low surfactant systems stabilized with associative and non-associative polymers can enhance the hydration of the skin and can potentially outperform other benefit agents in similar personal care compositions. As shown in Table 4, an enhancement in hydration can be observed 3 hours after the initial application of personal care compositions comprising sucrose polyesters (i.e. Examples A, D, and E) as shown by the increase in corneometer readings for all three sucrose polyester formulations (Examples A, D, and E) as compared to the no treatment control. Interestingly, treatment with personal care compositions comprising sucrose polyesters (Examples A, D, and E) resulted in higher corneometer readings at 3 hours after application as compared to Example C that comprised petrolatum (a known occlusive agent) and Example B that comprised soy bean oil. Surprisingly, treatment with the sucrose polyesters-containing Examples D and A resulted in a two-fold or three-fold increase in the corneometer readings as compared to Example C and Example B.

Even after 24 hours from the initial application, as shown in Table 5, treatment with any of the three sucrose polyester-containing Examples A, D, and E led to enhanced corneometer readings as compared to the no treatment control. Surprisingly, even treatment with 2 of the 3 sucrose polyester-containing samples (i.e. Examples D and E) resulted in either similar or higher corneometer readings as compared to treatment with the petrolatum-containing sample (i.e. Example C). Interestingly, all three sucrose polyester-containing samples (Examples A, D, and E) outperformed the non-sucrose polyester-containing Example B 24 hours after application as shown in Table 5 by the improvement in corneometer readings.

A. Structured Cleansing Phase

As noted herein, a personal care composition can include a structured cleansing phase and a benefit phase. The structured cleansing phase and the benefit phase can be in physical contact. The phases can be blended or mixed to a significant degree, but still be physically distinct such that the physical distinctiveness is undetectable to the naked eye. The phases can also be made to occupy separate and distinct physical spaces inside a package in which the phases can be stored. In such an arrangement, the structured cleansing phase and the benefit phase can be stored such that the phases are not in direct contact with one another. The structured cleaning phase and the benefit phase can be in physical contact while remaining visibly distinct to give, for example, a striped or marbled configuration.

The personal care composition can include a combination of one or more of the above multiphase personal care compositions. For example, one blended multiphase personal care composition can be stacked as stripes with another blended multiphase personal care composition.

The personal care composition can include a cleansing phase. The cleansing phase can comprise as least one anionic surfactant. The cleansing phase may contain from 3% to about 20%, from about 5% to about 15%, from about from about 7% to about 15%, from about 5% to about 13%, from about 5% to about 20%, or any combination of the upper, lower, and included limits within the ranges 2% to 30%, of surfactant, by weight of the personal care composition.

The cleansing phase may comprise a structured domain. The structured domain is preferably an opaque structured domain, which is preferably a lamellar phase. The lamellar phase can provide resistance to shear, adequate yield to suspend particles and droplets while providing long term stability because it is thermodynamically stable. The lamellar phase tends to have a viscosity that minimizes the need for viscosity modifiers, but they can be included if desired. The cleaning phase may comprise more than one surfactant.

The anionic surfactants can be either linear or branched. Examples of some suitable linear anionic surfactants include ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, sodium cocoyl isethionate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, and combinations thereof.

Examples of some suitable branched anionic surfactants include but are not limited to the following surfactants: sodium trideceth sulfate, sodium tridecyl sulfate, sodium $C_{12-13}$ alkyl sulfate, sodium $C_{12-15}$ alkyl sulfate, sodium $C_{11-15}$ alkyl sulfate, sodium $C_{12-18}$ alkyl sulfate, sodium $C_{10-16}$ alkyl sulfate, sodium $C_{12-13}$ pareth sulfate, sodium $C_{12-13}$ pareth-$n$ sulfate, sodium $C_{12-14}$ pareth-$n$ sulfate, and combinations thereof. Other salts of all the aforementioned surfactants are useful, such as TEA, DEA, ammonia, potassium salts. Useful alkoxylates include the ethylene oxide, propylene oxide and EO/PO mixed alkoxylates. Phosphates, carboxylates and sulfonates prepared from branched alcohols are also useful anionic branched surfactants. Branched surfactants can be derived from synthetic alcohols such as the primary alcohols from the liquid hydrocarbons produced by Fischer-Tropsch condensed syngas, for example Safol™ 23 Alcohol available from Sasol North America, Houston, TX; from synthetic alcohols such as Neodol™ 23 Alcohol available from Shell Chemicals, USA; from synthetically made alcohols such as those described in U.S. Patent No. 6,335,312 issued to Coffindaffer, et al on January 1, 2002. Suitable examples of alcohols are Safol™ 23 and Neodol™ 23. Suitable examples of alkoxylated alcohols are Safol™ 23-3 and Neodol™ 23-3. Sulfates can be prepared by conventional processes to high purity from a sulfur based $SO_3$ air stream process, chlorosulfonic acid process, sulfuric acid process, or Oleum process. Preparation via $SO_3$ air stream in a falling film reactor is a preferred sulfation process.

The anionic surfactant may also be STnS, wherein n can define average moles of ethoxylation. A structured cleansing phase can include from about 5% to about 20%, from about 7% to about 18%, from about 9% to about 16%, from about 11% to about 14%, by weight of the personal care composition, of STnS. A structured cleansing phase can include from 5% to 20%, from 7% to 18%, from 9% to 16%, from 11% to 14%, by weight of the personal care composition, of STnS. n can range from about 0 to about 3, from about 0.5 to about 2.7, from about 1.1 to about 2.5, from about 1.8 to about 2.2, or n can be about 2. When n is less than 3, STnS can provide improved stability, improved compatibility of benefit agents within the personal care compositions, and increased mildness of the personal care composition. Such described benefits of STnS are disclosed in U.S. Patent Application Serial No. 13/157,665.

Further, the structured cleansing phase can comprise a structuring system wherein the structuring system can comprise an associative polymer and a non-associative polymer. The structuring system can comprise from about 0.01% to about 5%, from about 0.05% to about 1%, from about 0.07% to about 0.5%, or from about 0.1% to about 0.3%, by weight of the personal care composition, of a non-associative polymer. The structuring system can also comprise from 0.01% to 5%, from 0.05% to 1%, from 0.07% to 0.5%, or from 0.1% to 0.3%, by weight of the personal care composition, of a non-associative polymer. The structuring system comprises from 0.01% to 0.03%, by weight of the personal care composition, of an associative polymer. As noted herein, stability of a personal care composition can be maintained or enhanced even with the reduction of associative polymer with the addition of a non-associative polymer.

Such associative polymers can be crosslinked, alkali swellable, associative polymers comprising acidic monomers and associative monomers with hydrophobic end groups, whereby the associative polymer comprises a percentage hydrophobic modification and a hydrophobic side chain comprising alkyl functional groups. Without intending to be limited by theory, it is believed the acidic monomers can contribute to an ability of the associative polymer to swell in water upon neutralization of acidic groups; and associative monomers anchor the associative polymer into structured surfactant hydrophobic domains, e.g., lamellae, to confer structure to the surfactant phase and keep the associative polymer from collapsing and losing effectiveness in the presence of an electrolyte. The crosslinked, associative polymer can comprise a percentage hydrophobic modification, which is a mole percentage of monomers expressed as a percentage of a total number of all monomers in a polymer backbone, including both acidic and other non-acidic monomers. Percentage hydrophobic modification of the associative polymer, hereafter %HM, can be determined by the ratio of monomers added during synthesis or by analytical techniques such as proton nuclear magnetic resonance (NMR). Associative alkyl side chains can comprise, for example, butyl, propyl, stearyl, steareth, cetyl, lauryl, laureth, octyl, behenyl, beheneth, steareth, or other linear, branched, saturated, or unsaturated alkyl or alketh hydrocarbon side chains.

It has also been discovered that crosslinked, associative polymers having certain %HM and certain carbon numbers of hydrophobic end groups of alkyl side chains can provide significant enhancement of structure to personal care compositions comprising a structured surfactant, especially to personal care compositions comprising reduced levels of surfactant. Such associative polymers can also provide the above structure at surprisingly low levels of polymer structurant. Concentrations of associative polymers of up to about 5% or even 10% have been known to provide a sufficient amount structure (e.g., exemplary compositions of U.S. Patent Nos. 7,119,059 (Librizzi, et al.) and 6,897,253 (Schmucker-Castner, et al.). It has been discovered that when an associative polymer %HM and an alkyl side chain number of carbons can be optimized, the structure of an aqueous structured surfactant phase can be increased using only less than about 3 wt%, less than about 2%, less than about 1%, and less than about 0.2%, of an associative polymer, as a percentage of an aqueous structured surfactant phase.

The acidic monomer can comprise any acid functional group, for example sulfate, sulfonate, carboxylate, phosphonate, or phosphate or mixtures of acid groups. The acidic monomer can comprise, for example, a carboxylate. Alternatively, the acidic monomer can be an acrylate, including acrylic acid and/or methacrylic acid. The acidic monomer can comprise a polymerizable structure, e.g., vinyl functionality. Mixtures of acidic monomers, for example acrylic acid and methacrylic acid monomer mixtures, may be useful as well.

The associative monomer can comprise a hydrophobic end group and a polymerizable component, e.g., vinyl, which can be attached. The hydrophobic end group can be attached to the polymerizable component, hence to the polymer chain, by different means but can be attached by an ether or ester or amide functionality, such as an alkyl acrylate or a vinyl alkanoate monomer. The hydrophobic end group can also be separated from the chain, for example, by an alkoxy ligand such as an alkyl ether. The associative monomer can be, for example, an alkyl ester, an alkyl (meth)acrylate, where (meth)acrylate is understood to mean either methyl acrylate or acrylate, or mixtures of the two.

Sometimes, the hydrophobic end group of the associative polymer can be incompatible with the aqueous phase of the personal care composition and can associate with lathering surfactant hydrophobe components. Without intending to be limited by theory, it is believed that longer alkyl chains of structuring polymer hydrophobe end groups can increase incompatibility with the aqueous phase to enhance structure, whereas shorter alkyl chains having carbon numbers closely resembling lathering surfactant hydrophobes (e.g., 12 to 14 carbons) or multiples thereof (for bilayers, e.g.) can also be effective. An ideal range of hydrophobic end group carbon numbers combined with an optimal percentage of hydrophobic monomers expressed as a percentage of the polymer backbone can provide increased structure to the personal care composition comprising a structured surfactant with low levels of polymer structurant.

An exemplary associative polymer can include AQUPEC® SER-300 made by Sumitomo Seika of Japan, which is an acrylate/$C_{10}$-$C_{30}$ alkyl acrylate cross-polymer and comprises stearyl side chains with less than about 1% HM. Associative polymers can comprise about $C_{16}$ (cetyl) alkyl hydrophobic side chains with about 0.7% hydrophobic modification, but a percentage hydrophobic modification can be up to an aqueous solubility limit in surfactant compositions (e.g., up to 2%, 5%, or 10%). Other associative polymers can include AQUPEC® SER-150 (acrylate/$C_{10}$-$C_{30}$ alkyl acrylate cross-polymer) comprising about $C_{18}$ (stearyl) side chains and about 0.4% HM.

As set forth above, the structured cleansing phase of a personal care composition can further include a non-associative polymer. Suitable non-associative polymers can include water-dispersible polymers with relatively uniform hydrophilic backbone lacking hydrophobic groups. Examples of non-associative polymers can include biopolymer polysaccharides (e.g., xanthan gum, gellan gum), cellulosic polysaccharides (e.g., carboxymethyl cellulose, carboxymethyl hydroxyethyl cellulose), other polysaccharides (e.g., guar gum, hydroxypropyl guar, and sodium alginate), and synthetic hydrocarbon polymers (e.g., polyacrylamide and copolymers, polyethylene oxide, polyacrylic acid copolymers).

Personal care compositions can additionally comprise an organic cationic deposition polymer in one or more phases as a deposition aid for the benefit agents described herein. Suitable cationic deposition polymers can contain cationic nitrogen-containing moieties such as quaternary moieties. Non-limiting examples of cationic deposition polymers can include polysaccharide polymers, such as cationic cellulose derivatives. Cationic cellulose polymers can be salts of hydroxyethyl cellulose reacted with trimethyl ammonium substituted epoxide, referred to in the industry (CTFA) as Polyquaternium 10, which can be available from Amerchol Corp. (Edison, N.J.) in their Polymer KG, JR, and LR series of polymers. Other suitable cationic deposition polymers can include cationic guar gum derivatives, such as guar hydroxypropyltrimonium chloride, specific examples of which can include the Jaguar series commercially available from Rhodia Inc. and N-Hance polymer series commercially available from Aqualon. Deposition polymers can have a cationic charge density from about 0.8 meq/g to about 2.0 meq/g or from about 1.0 meq/g to about 1.5 meq/g.

The personal care composition can be optionally free of or substantially free of sodium lauryl sulfate, hereinafter SLS, and/or ammonium lauryl sulfate, hereinafter ALS, and can comprise at least a 70% lamellar structure. However, in an alternative arrangement, the structured cleansing phase can comprise at least one surfactant, wherein the at least one surfactant includes SLS and/or ALS. Suitable examples of SLS are described in U.S. Patent Application Serial No. 12/817,786.

A personal care composition can further comprise from about 0.1% to about 20%, by weight of the personal care composition, of a cosurfactant. The cosurfactant can comprise amphoteric surfactants, zwitterionic surfactants, or mixtures thereof. For example, a personal care composition can include an amphoteric surfactant and/or a zwitterionic surfactant. Suitable amphoteric or zwitterionic surfactants can include those described in U.S. Patent Nos. 5,104,646 and 5,106,609.

Amphoteric surfactants can include those that can be broadly described as derivatives of aliphatic secondary and tertiary amines in which an aliphatic radical can be a straight or branched chain and wherein an aliphatic substituent can contain from about 8 to about 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and products described in U.S. Pat. No. 2,528,378. Other examples of amphoteric surfactants can include sodium lauroamphoacetate, sodium cocoamphoactetate, disodium lauroamphoacetate disodium cocodiamphoacetate, and mixtures thereof. Amphoacetates and diamphoacetates can also be used.

Zwitterionic surfactants suitable for use can include those that are broadly described as derivatives of aliphatic quaternary ammonium, phosphonium, and sulfonium compounds, in which aliphatic radicals can be straight or branched chains, and wherein an aliphatic substituent can contain from about 8 to about 18 carbon atoms such that one carbon atom can contain an anionic group, e.g., carboxy, sulfonate, sulfate, phosphate, or phosphonate. Other zwitterionic surfactants can include betaines, including cocoamidopropyl betaine.

Other suitable surfactants or cosurfactants that can generally be used in a structured cleansing phase for a personal care composition are described in McCutcheon's: Detergents and Emulsifiers North American Edition (Allured Publishing Corporation 1947) (1986), McCutcheon's, Functional Materials North American Edition (Allured Publishing Corporation 1973) (1992) and U.S. Patent No. 3,929,678 (filed Aug. 1, 1974).

The structured cleansing phase of the personal care composition can also comprise water. The structured cleansing phase of the personal care composition can comprise from about 10% to about 90%, from about 40% to about 85%, or from about 60% to about 80%, by weight of the personal care composition, of water.

Other optional additives can be included in the cleaning phase, including, for example, an emulsifier (e.g., non-ionic emulsifier) and electrolyes. Suitable electrolytes can include anions such as phosphate, chloride, sulfate, citrate, and mixtures thereof and cations such as sodium, ammonium, potassium, magnesium, and mixtures thereof. For example, suitable electrolytes can include sodium chloride, ammonium chloride, sodium sulfate, ammonium sulfate, and mixtures thereof. Other suitable emulsifiers and electrolytes are described in U.S. Patent Application Serial No. 13/157,665.

B. Benefit Phase

As noted herein, personal care compositions can include a benefit phase. The benefit phase can be hydrophobic and/or anhydrous. The benefit phase can also be substantially free of or free of surfactant.

The benefit phase can also include one or more benefit agents. In particular, the benefit phase can comprise from about 0.1% to about 50%, by weight of the personal care composition, of a benefit agent. In other arrangements, the benefit phase can include from about 0.5% to about 20%, by weight of the personal care composition, of the benefit agent. Examples of such benefit agents can include petrolatum, glyceryl monooleate, mineral oil, natural oils (e.g., soybean oil), and mixtures thereof.

Benefit agents can include water insoluble or hydrophobic benefit agents. Additional examples of benefit agents can include SEFOSE®, lanolin, lanolin derivatives, lanolin esters, lanolin oil, natural waxes, synthetic waxes, volatile organosiloxanes, derivatives of volatile organosiloxanes, non-volatile organosiloxanes, derivatives of non-volatile organosiloxanes, natural triglycerides, synthetic triglycerides, and combinations thereof. Other suitable benefit agents are described in U.S. Patent Application Serial No. 13/157,665.

SEFOSE® includes one or more types of sucrose polyesters. Sucrose polyesters are derived from a natural resource and therefore, the use of sucrose polyesters as the benefit agent can result in a positive environmental impact. Sucrose polyesters are polyester materials having multiple substitution positions around the sucrose backbone coupled with the chain length, saturation, and derivation variables of the fatty chains. Such sucrose polyesters can have an esterification ("IBAR") of greater than about 5. For example, the sucrose polyester may have an IBAR of about 5 to about 8. In another example, the sucrose polyester may have an IBAR of about 5-7; in another example, the sucrose polyester can have an IBAR of about 6. In yet another example, the sucrose polyester can have an IBAR of about 8. As sucrose polyesters can be derived from natural resources, a distribution in the IBAR and chain length may exist. For example, a sucrose polyester having an IBAR of 6 may contain a mixture of mostly IBAR of about 6, with some IBAR of about 5, and some IBAR of about 7. Additionally, such sucrose polyesters may have a saturation or iodine value ("IV") of about 3 to about 140. In another example, the sucrose polyester may have an IV of about 10 to about 120. In yet another example, the sucrose polyester may have an IV of about 20 to 100. Further, such sucrose polyesters may have a chain length of about $C_{12}$ to $C_{20}$.

Non-limiting examples of sucrose polyesters suitable for use include SEFOSE® 1618S, SEFOSE® 1618U, SEFOSE® 1618H, Sefa Soyate IMF 40, Sefa Soyate LP426, SEFOSE® 2275, SEFOSE® C1695, SEFOSE® C18:0 95, SEFOSE® C1495, SEFOSE® 1618H B6, SEFOSE® 1618S B6, SEFOSE® 1618U B6, Sefa Cottonate, SEFOSE® C1295, Sefa C895, Sefa C1095, SEFOSE® 1618S B4.5, all available from The Procter and Gamble Co. of Cincinnati, Ohio. Sucrose polyesters can also be combined with other benefit agents in the benefit phase.

Non-limiting examples of glycerides suitable for use as hydrophobic benefit agents herein can include castor oil, safflower oil, corn oil, walnut oil, peanut oil, olive oil, cod liver oil, almond oil, avocado oil, palm oil, sesame oil, soybean oil, vegetable oils, sunflower seed oil, vegetable oil derivatives, coconut oil and derivatized coconut oil, cottonseed oil and derivatized cottonseed oil, jojoba oil, cocoa butter, petrolatum, mineral oil, and combinations thereof.

Non-limiting examples of alkyl esters suitable for use as hydrophobic benefit agents herein can include isopropyl esters of fatty acids and long chain esters of long chain (i.e. $C_{10}$-$C_{24}$) fatty acids, e.g., cetyl ricinoleate, non-limiting examples of which can include isopropyl palmitate, isopropyl myristate, cetyl riconoleate, and stearyl riconoleate. Other examples can include hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use as hydrophobic benefit agents herein can include oleyl myristate, oleyl stearate, oleyl oleate, and combinations thereof.

Non-limiting examples of polyglycerin fatty acid esters suitable for use as hydrophobic benefit agents herein can include decaglyceryl distearate, decaglyceryl diisostearate, decaglyceryl monomyriate, decaglyceryl monolaurate, hexaglyceryl monooleate, and combinations thereof.

Non-limiting examples of lanolin and lanolin derivatives suitable for use as hydrophobic benefit agents herein can include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate, and combinations thereof.

Non-limiting examples of silicone oils suitable for use as hydrophobic benefit agents herein can include dimethicone copolyol, dimethylpolysiloxane, diethylpolysiloxane, mixed $C_1$-$C_{30}$ alkyl polysiloxanes, phenyl dimethicone, dimethiconol, and combinations thereof. Non-limiting examples of silicone oils useful herein are described in U.S. Patent No. 5,011,681. Still other suitable hydrophobic skin benefit agents can include milk triglycerides (e.g., hydroxylated milk glyceride) and polyol fatty acid polyesters.

Additional optional materials can also be added to the personal care composition to treat the skin, or to modify the aesthetics of the personal care composition as is the case with perfumes, colorants, dyes, or the like. Optional materials useful in products herein can be categorized or described by their cosmetic and/or therapeutic benefit or their postulated mode of action or function. However, it can be understood that actives and other materials useful herein can, in some instances, provide more than one cosmetic and/or therapeutic benefit or function or operate via more than one mode of action. Therefore, classifications herein can be made for convenience and cannot be intended to limit a material to a particularly stated application or applications listed. A precise nature of these optional material and levels of incorporation thereof, will depend on the physical form of the personal care composition and the nature of the cleansing operation for which it is to be used. Optional materials can usually be formulated at about 6% or less, about 5% or less, about 4% or less, about 3% or less, about 2% or less, about 1% or less, about 0.5% or less, about 0.25% or less, about 0.1% or less, about 0.01% or less, or about 0.005% or less by weight of the personal care composition.

To further improve stability under stressful conditions such as high temperature and vibration, the densities of the separate phases can be adjusted such that they can be substantially equal. To achieve this, low density microspheres can be added to one or more phases of the personal care composition. Examples of personal care compositions that comprise low density microspheres are described in a patent application published on May 13, 2004 under U.S. Patent Publication No. 2004/0092415A1 entitled "Striped Liquid Personal Cleansing Compositions Containing A Cleansing Phase and A Separate Phase with Improved Stability," filed on Oct. 31, 2003 by Focht, et al.

The personal care composition can also comprise a benefit component that can be selected from the group consisting of thickening agents; preservatives; antimicrobials; fragrances; chelators (e.g., such as those described in U.S. Pat. No. 5,487,884 issued to Bisset, et al.); sequestrants; vitamins (e.g., Retinol); vitamin derivatives (e.g., tocophenyl actetate, niacinamide, panthenol); sunscreens; desquamation actives (e.g., such as those described in U.S. Pat. No. 5,681,852 and 5,652,228 issued to Bisset); anti-wrinkle/ anti-atrophy actives (e.g., N-acetyl derivatives, thiols, hydroxyl acids, phenol); anti-oxidants (e.g., ascorbic acid derivatives, tocophenol) skin soothing agents/skin healing agents (e.g., panthenoic acid derivatives, aloe vera, allantoin); skin lightening agents (e.g., kojic acid, arbutin, ascorbic acid derivatives) skin tanning agents (e.g., dihydroxyacteone); anti-acne medicaments; essential oils; sensates; pigments; colorants; pearlescent agents; interference pigments (e.g., such as those disclosed in U.S. Pat. No. 6,395,691 issued to Liang Sheng Tsaur, U.S. Pat. No. 6,645,511 issued to Aronson, et al., U.S. Pat. No. 6,759,376 issued to Zhang, et al, U.S. Pat. No. 6,780,826 issued to Zhang, et al.) particles (e.g., talc, kolin, mica, smectite clay, cellulose powder, polysiloxane, silicas, carbonates, titanium dioxide, polyethylene beads) hydrophobically modified non-platelet particles (e.g., hydrophobically modified titanium dioxide and other materials described in a commonly owned, patent application published on Aug. 17, 2006 under Publication No. 2006/0182699A, entitled "Personal Care Compositions Containing Hydrophobically Modified Non-platelet particle filed on Feb. 15, 2005 by Taylor, et al.) and mixtures thereof. The personal care compositions can comprise from about 0.1% to about 4%, by weight of the personal care composition, of hydrophobically modified titanium dioxide. Other such suitable examples of such skin actives are described in U.S. Patent Application Serial No. 13/157,665.

Other optional materials can be those materials approved for use in cosmetics and that are described in the CTFA Cosmetic Ingredient Handbook, Second Edition, The Cosmetic, Toiletries, and Fragrance Association, Inc. 1988, 1992.

III. Methods Relating to the Use of Personal Care Compositions

The personal care compositions as described herein can provide a multitude of benefits. For example, a method for increasing stability in a personal care composition can include forming the personal care composition with a structured cleansing phase having an associative polymer and a non-associative polymer. The personal care composition can be any of the personal care compositions as described herein. By forming a personal care composition with an associative polymer and a non-associative polymer, the stability can be increased relative to that for Comparative Examples 1-3 shown in Table 1 below, each of which does not include a non-associative polymer. As shown in Table 2, a viscosity change for each of Inventive Examples 4-6 is less than about 30% from an initial product viscosity to a product viscosity after ten days at 50°C.

Personal care compositions as described herein can also provide enhanced deposition of a benefit agent to the hair and/or skin of a consumer. One method for enhancing deposition of a benefit agent can include forming a personal care composition with a structured cleansing phase and benefit phase and placing the personal care composition onto the skin and/or hair of a consumer. Such a structured cleansing phase can include an associative polymer and a non-associative polymer and the benefit phase can include a benefit agent. The personal care composition can be any of the personal care compositions as described herein. Methods for depositing benefit agents using personal care compositions are further described in U.S. Patent Application Serial No. 12/510,880.

The personal care compositions as described herein can also provide enhanced hydration of the skin and/or hair of consumer. One method for enhancing the hydration of the skin and/or hair can include forming a personal care composition with a structured cleansing phase and a benefit phase comprising sucrose polyesters and placing the personal care composition onto the skin and/or hair of a consumer. Such a structured cleansing phase can include an associative polymer and a non-associative polymer. The personal care composition can be any of the personal care compositions as described herein. Tables 4 and 5 provide examples of an enhanced hydration of the skin when a personal care composition comprising sucrose polyesters is applied to the skin.

IV. Test Methods

A. T-Bar Viscosity Method

The viscosity of a personal care composition can be assessed by the T-Bar Viscosity Method. The apparatus for T-Bar measurements includes a Brookfield DV-II+ Pro Viscometer with Helipath Accessory; a chuck, weight and closer assembly for T-bar attachment; a T-bar Spindle D, a personal computer with Rheocalc software from Brookfield, and a cable connecting a Brookfield Viscometer to a computer. First, weigh 80 grams of a personal care composition in a 4-oz. glass jar. Measure a T-bar viscosity by carefully dropping the T-Bar Spindle to an interior bottom of the glass jar and set the Helipath stand to travel in an upward direction. Open the Rheocalc software and set the following data acquisition parameters: Speed to 5 rpm, Time Wait for Torque to 00:01 (1 second), and Loop Start Count to 100. Start data acquisition and turn on the Helipath stand to travel upward at a speed of 22 mm/minute. The T-Bar viscosity is an average T-Bar viscosity reading between the 10th reading and the 90th reading (the first ten readings and the last ten readings are not used for the average T-Bar viscosity calculation). The T-Bar viscosity reading is provided in cP. After obtaining the initial viscosity reading, place the personal care composition at 50°C for 10 days for rapid aging. After finishing the stability testing at 50°C, the sample is equilibrated at 25°C for 24 hours. Then repeat viscosity measurement to obtain final viscosity. Measure percent change of the initial viscosity from the final viscosity measurement to obtain the percent change in viscosity.

B. Zero Shear Viscosity and Young's Modulus Methods

The Zero Shear Viscosity of a material which is a phase or a component of the personal care composition, can be measured either prior to combining in the personal care composition, after preparing a composition, or first separating a phase or component from a personal care composition by suitable physical separation means, such as centrifugation, pipetting, cutting away mechanically, rinsing, filtering, or other separation means.
A controlled stress rheometer such as a TA Instruments AR2000 Rheometer is used to determine the Zero Shear Viscosity. The determination is performed at 25°C with a 4 cm diameter parallel plate measuring system and a 1 mm gap. The geometry has a shear stress factor of 79580 m-3 to convert torque obtained to stress. Serrated plates can be used to obtain consistent results when slip occurs.
First, material is positioned on a rheometer base plate; the measurement geometry (upper plate) is moved into position 1.1 mm above the base plate. Excess material at the geometry edge is removed by scraping after locking the geometry. The geometry is then moved to the target 1 mm position above the base plate and a pause of about 2 minutes is allowed to allow loading stresses to relax. This loading procedure ensures no tangential stresses are loaded at the measurement onset which can influence the results obtained. If the material comprises particles discernible to the eye or by feel (e.g., beads) that are larger than about 150 microns in number average diameter, the gap setting between the base plate and upper plate is increased to the smaller of 4 mm or 8-fold the diameter of the 95th volume percentile particle diameter. If a phase has any particle larger than 5 mm in any dimension, the particles are removed prior to the measurement.
The measurement is performed by applying a continuous shear stress ramp from 0.1 Pa to 1,000 Pa over a time interval of 4 minutes using a logarithmic progression, i.e., measurement points evenly spaced on a logarithmic scale. Thirty measurement points per decade of stress increase are obtained. If the measurement result is incomplete, for example, if material is observed to flow from the gap, results obtained are evaluated with incomplete data points excluded. If there are insufficient points to obtain an accurate measurement, the measurement is repeated with increased number of sample points.
The Young's Modulus (Pa) is obtained by graphing Stress (Pa) vs. Strain (unitless) and obtaining a slope of a regression line of an initial linear region between Stress vs. Strain, typically occurring in the region below about 4% strain. If the relationship is not linear, the linear regression line slope below 2% strain is taken as the Young's Modulus (Pa), using unitless strain.
The Zero Shear Viscosity is obtained by taking a first median value of viscosity in Pascal-seconds (Pa-s) for viscosity data obtained between and including 0.1 Pa and a point where viscosity begins to steeply decline. After taking the first median viscosity, all viscosity values greater than 5-fold the first median value and less than 0.2 x the median value are excluded, and a second median viscosity value is obtained of the same viscosity data, excluding the indicated data points. The second median viscosity so obtained is the Zero Shear Viscosity.
As set forth above, a structured cleansing phase can be considered to be structured if the structured cleansing phase has a Zero Shear Viscosity of about 200 Pa-s or more, about 500 Pa-s or more, about 1,000 Pa-s or more, about 1,500 Pa-s or more, or about 2,000 Pa-s or more.

C. Third-Phase Method for Determining Structured Surfactant Stability

A Third-Phase Method can be used to determine structured surfactant phase stability in a personal care composition. The method involves placing the personal care compositions at 50°C for 10 days for rapid aging. After rapid aging, transfer about 4 grams of the personal care composition into a Beckman Centrifuge Tube (11 mm x 60 mm). Place the centrifuge tube in a Beckman LE-80 Ultracentrifuge and operate the ultracentrifuge under the following conditions: 50,000 rpm, 2 hours, and 40°C.

After ultracentrifugation, determine the third-phase volume by measuring a height for each of various surfactant phases using an Electronic Digital Caliper (within 0.01 mm).

A very top layer is a hydrophobic benefit phase layer (e.g., hydrocarbons or soybean oil). The layers below the hydrophobic benefit phase layers containing surfactant/water can determined by the following: $H_a$ is a height of all layers containing surfactant/water and $H_b$ is a height of a clear "third-phase" layer just below the hydrophobic benefit phase layer. It is important to record the readings within 30 minutes after the ultracentrifugation is finished to minimize material migration across different layers. The third phase volume is calculated as:

$$\text{Third-Phase Volume}\% = H_b/H_a *100\%.$$

A personal care composition comprising a structured surfactant can comprises less than about 10% "third-phase" volume, less than about 5% "third-phase" volume, less than about 2% "third-phase" volume, or less than about 1% "third-phase" volume after rapid aging stability protocol. In another arrangement, the personal care composition comprising a structured surfactant can comprise about 0% "third-phase" volume after rapid aging protocol.

D. Ultracentrifugation Method

The Ultracentrifugation Method is a physical method used to determine an amount of structure in a personal care composition or a subset of a personal care composition. The method can also be used to determine a rate at which a personal care composition comprising a structured surfactant dissolves upon dilution to present effective amounts of surfactant to a cleaning environment proximal to surfaces.

A personal care composition can be separated by ultracentrifuge into separate but distinguishable layers. The personal care composition can have multiple distinguishable layers (e.g., a structured surfactant layer and a benefit layer).

First, dispense about 4 grams of the personal care composition into a Beckman Centrifuge Tube (11 mm x 60 mm) to fill the tube. Next, dilute the personal care composition to a 10% Dilution Level using 90% of the personal care composition and 10% DI water using an appropriate mixer and dispense an equal amount of the personal care composition into a companion centrifuge tube. Continue to dilute the personal care composition and fill tubes in the same manner until a 60% Dilution Level is obtained for the personal care composition using 40% of the personal care composition with 60% DI water. Place the centrifuge tubes in an ultracentrifuge (Beckman Model L8-M or equivalent) using a sling rotor and ultracentrifuge using the following conditions: 50,000 rpm, 2 hours, and 40°C.

Measure relative phase volumes for each phase of the personal care composition by measuring a height for each layer using an Electronic Digital Caliper (within 0.01mm). Layers are identified by those skilled in the art by physical observation techniques paired with chemical identification if needed. For example, the structured surfactant layer can be identified by transmission electron microscopically (TEM), polarized light microscopy, and/or X-ray diffraction as a structured lamellar phase comprising multilamellar vesicles, and the hydrophobic benefit layer can be identified by its low moisture content (less than about 10% water as measured by Karl Fischer Titration). Measure a total height Ha, which includes all materials in the ultracentrifuge tube. Next, the height of each layer is measured from a bottom of the centrifuge tube to a top of the layer, and the span of each layer can be algebraically determined by subtraction. The benefit layer may comprise several layers. If the benefit phase splits, a sum of the benefit layers measured is the benefit layer height, Hb. Generally, a hydrophobic benefit layer, when present, is at a top of the centrifuge tube.

The surfactant phase may comprise several layers or a single layer, Hc. There may also be a micellar, unstructured, clear isotropic layer at the bottom or next to the bottom of the ultracentrifuge tube. Layers immediately above the isotropic phase generally comprise higher surfactant concentration with higher ordered structures (such as liquid crystals). Such structured layers can be opaque to naked eyes, or translucent, or clear. If several structured layers are present, Hc is a sum of individual structured layers. If any type of polymer-surfactant phase is present, it is considered a structured phase and included in the measurement of Hc. A sum of aqueous phases is Hs.

Finally, the structured domain volume ratio is calculated as follows:

$$\text{Structured Domain Volume Ratio} = \ Hc \ / \ Hs \ *100\%$$

If there is no benefit phase present, use the total height as the surfactant layer height, Hs = Ha. The Structured Domain Volume Ratio is the Lamellar Phase %. The measurement is made for each dilution prepared and centrifuged (i.e., the Structured Domain Volume Ratio is determined for the composition, and for 90%, 80%, 70% and 60% dilutions prepared as indicated above).

The highest amount of dilution (i.e., the lowest Dilution Level) wherein the personal care composition maintains at least 95% Lamellar Phase % is an indicator of amount of structure for personal care compositions having varying n values for STnS.

The highest dilution wherein the personal care composition has at least 95% lamellar phase is greater than about 15% , greater than about 25%, or greater than about 35%.

The personal care composition has a Structured Domain Volume Ratio of greater than about 40%, greater than about 45%, greater than about 50%, greater than about 55%, greater than about 60%, greater than about 65%, greater than about 70%, greater than about 75%, greater than about 80%, greater than about 85%, or greater than about 90%, by volume of personal care composition comprising an aqueous surfactant.

V. Examples

A. Examples 1-3

For Comparative Examples 1-3, personal care compositions are formed with an associative polymer. Compositional information with respect to Comparative Examples 1-3 can be found in Table 1. Comparative Examples 1-3 can be prepared through conventional mixing methods. First, a polymer-TDA-3 premix can be prepared by adding AQUPEC® SER 300 polymer into tricedeth-3 (Iconal TDA-3 ethoxylated tridecyl alcohol). Water, sodium chloride, N-Hance CG-17 polymer, and cocamidopropyl betaine can be added to the main mixing vessel with continuous mixing. Then, AQUPEC®-polymer-TDA-3 premix and sodium trideceth sulfate can be added, and the pH can be adjusted to about 5.7 using citric acid. Then preservatives and perfume can be added to the vessel, which can be mixed until homogeneous. The structured surfactant composition can be blended with the hydrophobic benefit agent (soybean oil) through a SpeedMixer™ (Model DAC 400 from FlackTek Inc, Landrum SC) at 2000 rpm for 2 minutes. The Comparative Examples are considered to be unstable as the viscosity change for each Comparative Example is greater than about 30% from the initial product viscosity to the product viscosity after ten days at 50°C.

Table 1

| Ingredient / Property | Comparative Example 1 | Comparative Example 2 | Comparative Example 3 |
|---|---|---|---|
| Sodium Tridecyl Ether Sulfate (anioinic surfactant) | 9.27 | 9.27 | 9.27 |
| Cocamidopropyl Betaine (cosurfactant) | 2.27 | 2.27 | 2.27 |
| Sodium Chloride | 4.75 | 4.75 | 4.75 |
| PEG-90M | | | |
| Iconol TDA3-Ethoxylated Tridecyl Alcohol | 1.40 | 1.40 | 1.40 |
| N-Hance CG17 Cationic Guar (water soluble cationic polymer) | 0.42 | 0.42 | 0.42 |
| AQUPEC® SER 300C (associative polymer) | 0.06 | 0.08 | 0.10 |
| Water, Preservatives, Perfume | Q.S. | Q.S. | Q.S. |
| 100 Proc RBD Soybean Oil (hydrophobic benefit agent) | 10.0 | 10.0 | 10.0 |
| | | | |
| Product pH | 5.7 | 5.7 | 5.7 |
| Initial Product Viscosity, cP | 43315 | 49650 | 52770 |

(continued)

| Ingredient / Property | Comparative Example 1 | Comparative Example 2 | Comparative Example 3 |
|---|---|---|---|
| Product Viscosity (after 10 days @50°C), cP | 10155 | 12340 | 11855 |
| Viscosity Change | -77% | -75% | -78% |

B. Examples 4-6

For Inventive Examples 1-3, personal care compositions are formed with an associative polymer and a non-associative polymer. Inventive Examples 1-3 can be prepared through conventional mixing methods. First, a polymer-TDA-3 premix can be prepared by adding AQUPEC® SER 300 polymer, xanthan gum, and PEG-90M into tricedeth-3 (Iconal TDA-3 ethoxylated tridecyl alcohol). In the main mixing vessel, water, sodium chloride, N-Hance CG-17 polymer, cocamidopropyl betaine can be added with continuous mixing. Then, AQUPEC®-polymer-TDA-3 premix and sodium trideceth sulfate can be added, and the pH can be adjusted to about 5.7 using citric acid. Then preservatives and perfume can be added to the vessel, which can be mixed until homogeneous. The structured surfactant composition can be blended with the hydrophobic benefit agent (soybean oil) through a SpeedMixer™ (Model DAC 400 from FlackTek Inc, Landrum SC) at 2000 rpm for 2 minutes. Compositional information with respect to Inventive Examples 1-3 can be found in Table 2. The Inventive Examples are considered to be stable as the viscosity change for each Comparative Example is less than about 30% from the initial product viscosity to the product viscosity after ten days at 50°C.

Table 2

| Ingredient | Inventive Example 1 | Inventive Example 2 | Inventive Example 3 |
|---|---|---|---|
| Sodium Tridecyl Ether Sulfate (anioinic surfactant) | 9.27 | 9.27 | 9.27 |
| Cocamidopropyl Betaine (cosurfactant) | 2.27 | 2.27 | 2.27 |
| Sodium Chloride | 4.75 | 4.75 | 4.75 |
| PEG-90M | 0.1 | 0.1 | 0.1 |
| Iconol TDA3-Ethoxylated Tridecyl Alcohol | 1.48 | 1.48 | 1.48 |
| N-Hance CG17 Cationic Guar (water soluble cationic polymer) | 0.48 | 0.48 | 0.48 |
| AQUPEC® SER 300C (associative polymer) | 0.01 | 0.02 | 0.03 |
| Xanthan Gum (non-associative polymer) | 0.22 | 0.22 | 0.22 |
| Water, Preservatives, Perfume | Q.S. | Q.S. | Q.S. |
| 100 Proc RBD Soybean Oil (hydrophobic benefit agent) | 10.0 | 10.0 | 10.0 |
| | | | |
| Product pH | 5.7 | 5.7 | 5.7 |
| Initial Product Viscosity, cP | 27910 | 31960 | 36755 |
| Product Viscosity (after 10 days @50°C), cP | 23890 | 26205 | 27670 |
| Viscosity Change | -14% | -18% | -25% |

Table 3

| Surfactant Vanilla Base Compositions (Surfactant VB) | Example A (w/w%) | Example B (w/w%) | Example C (w/w%) | Example D (w/w%) | Example E (w/w%) |
|---|---|---|---|---|---|
| Sodium Trideceth-2 Sulfate | 6.48 | 6.37 | 6.48 | 6.48 | 9.05 |

(continued)

| Surfactant Vanilla Base Compositions (Surfactant VB) | Example A (w/w%) | Example B (w/w%) | Example C (w/w%) | Example D (w/w%) | Example E (w/w%) |
|---|---|---|---|---|---|
| Cocamidopropyl betaine | 1.94 | 1.91 | 1.94 | 1.94 | 2.7 |
| Trideceth-3 | 1.03 | 1.02 | 1.03 | 1.03 | 1.44 |
| Guar Hydroxypropyltrimonium chloride | 0.33 | 0.33 | 0.33 | 0.33 | 0.1 |
| Acrylates/C10-C30 alkylacrylates cross polymer (Aqupec SER 300) | 0.02 | 0.02 | 0.02 | 0.02 | 0.03 |
| Sodium Chloride | 4.65 | 4.65 | 4.65 | 4.65 | 4.65 |
| Xanthan Gum | 0.15 | 0.15 | 0.15 | 0.15 | 0.21 |
| Sefa Soyate | 1.0 | - | - | 0.9 | 1.0 |
| Soybean Oil | - | 1.80 | - | - | - |
| Glyceryl Monooleate | - | 0.20 | 0.1 | 0.1 | - |
| Petrolatum | - | - | 1.9 | - | - |
| Citric acid/sodium hydroxide | pH=5.7 | pH=5.7 | pH=5.7 | pH=5.7 | pH=5.7 |
| Water, perfume, preservatives | Q.S. | Q.S. | Q.S. | Q.S. | Q.S. |

C. Examples A-E

A surfactant vanilla base composition of Table 4 or Table 5 can be prepared by first premixing the Aqupec polymer with Trideceth-3. Add water, guar hydroxypropyltrimonium chloride, sodium chloride while mixing. Then, add sodium trideceth-2 sulfate, cocamidopropyl betaine, and the trideceth-3/Aqupec premix. Then add citric to adjust pH to 5.7. Then, add preservatives, and perfume. Keep mixing until homogeneous. Then, add the benefit agent(s) into the surfactant phase using a SpeedMixer and mix for 1 min at 2,000 rpm.

Table 4

| Corneometer 3 hours after one application | Sample Size | Treatment | Change in Adjusted Mean | Improvement vs. No Treatment Control |
|---|---|---|---|---|
| | 28 | No Treatment Control | 0.48 | - |
| | 29 | Example B | 1.28 | + 0.80 |
| | 27 | Example C | 1.51 | + 1.03 |
| | 27 | Example E | 1.98 | + 1.50 |
| | 27 | Example A | 3.02 | + 2.54 |
| | 30 | Example D | 3.20 | + 2.71 |

Table 5

| Corneometer 24 hours after one application | Sample Size | Treatment | Change in Adjusted Mean | Improvement vs. No Treatment Control |
|---|---|---|---|---|
| | 28 | No Treatment Control | -0.60 | - |
| | 29 | Example B | -0.21 | + 0.39 |

(continued)

| Corneometer 24 hours after one application | Sample Size | Treatment | Change in Adjusted Mean | Improvement vs. No Treatment Control |
|---|---|---|---|---|
| | 27 | Example A | 0.61 | + 1.21 |
| | 27 | Example C | 0.90 | + 1.50 |
| | 30 | Example D | 0.94 | + 1.54 |
| | 27 | Example E | 1.74 | + 2.34 |

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A personal care composition comprising:

    a) a structured cleansing phase comprising:

        i) from 5 % to 20 %, by weight of the personal care composition, of sodium trideceth (n) sulfate where n is between 0.5 and 2.7;
        ii) an amphoteric surfactant, a zwitterionic surfactant, or a combination thereof; and
        iii) a structuring system comprising from 0.01 % to 0.03 %, by weight of the personal care composition, of an associative polymer and from 0.01 % to 5 %, by weight of the personal care composition, of a non-associative polymer, wherein the associative polymer comprises acrylate/C10-C30 alkyl acrylate cross-polymers; and

    b) a benefit phase comprising from 0.1 % to 50 %, by weight of the personal care composition, of a benefit agent;

    wherein the structured cleansing phase and the benefit phase are in physical contact.

2. The personal care composition of claim 1, wherein the benefit agent is selected from the group consisting of sucrose polyesters; petrolatum; mineral oil; soybean oil; lanolin; lanolin derivatives; lanolin esters; lanolin oil; natural waxes; synthetic waxes; volatile organosiloxanes; derivatives of volatile organosiloxanes; non-volatile organosiloxanes; derivatives of non-volatile organosiloxanes; natural triglycerides; synthetic triglycerides; and combinations thereof.

3. The personal care composition of any preceding claim, wherein n is between 1.1 and 2.5, or preferably where n is 2.

4. The personal care composition of any preceding claim, wherein the benefit phase is free of surfactant.

5. The personal care composition of any preceding claim, wherein the structured cleansing phase comprises a zwitterionic surfactant.

6. The personal care composition of any preceding claim, wherein the structuring system further comprises an electrolyte.

7. The personal care composition of claim 6, wherein the electrolyte comprises an anion selected from the group consisting of phosphate, chloride, sulfate, citrate, and mixtures thereof; and a cation selected from the group consisting of sodium, ammonium, potassium, magnesium, and mixtures thereof.

8. The personal care composition of claim 6, wherein the electrolyte is selected from the group consisting of sodium chloride, ammonium chloride, sodium sulfate, ammonium sulfate, and mixtures thereof.

9. The personal care composition of any preceding claim, wherein the non-associative polymer is selected from the group consisting of polysaccharides, synthetic hydrocarbon polymers, and combinations thereof.

10. The personal care composition of claim 9, wherein the polysaccharide comprises xanthan gum.

11. The personal care composition of any preceding claim, wherein the structured cleansing phase comprises from 7 % to 18 %, preferably from 9 % to 16 %, or more preferably from 11 % to 14 %, by weight of the personal care composition, of sodium trideceth (n) sulfate where n is between 0.5 and 2.7.

12. The personal care composition of any preceding claim, wherein the structuring system comprises from 0.05 % to 1 %, preferably from 0.07 % to 0.5 %, or more preferably from 0.1 % to 0.3 %, by weight of the personal care composition, of the non-associative polymer.


**Patentansprüche**

1. Körperpflegezusammensetzung, umfassend:

   a) eine strukturierte Reinigungsphase, umfassend:

   i) von 5 bis 20 Gew.-%, bezogen auf das Gesamtgewicht der Körperpflegezusammensetzung, Natriumtrideceth-(n)-Sulfat, wobei n zwischen 0,5 und 2,7 liegt;
   ii) ein amphoteres Tensid, ein zwitterionisches Tensid oder eine Mischung davon; und
   iii) ein strukturierendes System, das von 0,01 bis 0,03 Gew.-%, bezogen auf das Gewicht der Körperpflegezusammensetzung, ein assoziatives Polymer und von 0,01 bis 5 Gew.-%, bezogen auf das Gewicht der Körperpflegezusammensetzung, ein nichtassoziatives Polymer umfasst, wobei das assoziative Polymer Acrylat/C10-C30 Alkylacrylat-Crosspolymere umfasst; und

   b) eine Nutzphase, die von 0,1 bis 50 Gew.-%, bezogen auf das Gewicht der Körperpflegezusammensetzung, einen Wirkstoff umfasst;

   wobei die strukturierte Reinigungsphase und die Nutzphase in gegenseitigem physikalischen Kontakt stehen.

2. Körperpflegezusammensetzung nach Anspruch 1, wobei der Wirkstoff ausgewählt ist aus der Gruppe bestehend aus Saccharosepolyestern; Petrolatum; Mineralöl; Sojabohnenöl; Lanolin; Lanolinderivaten; Lanolinestern; Lanolinöl; natürlichen Wachsen; synthetischen Wachsen; flüchtigen Organosiloxanen; Derivaten von flüchtigen Organosiloxanen; nichtflüchtigen Organosiloxanen; Derivaten von nichtflüchtigen Organosiloxanen; natürlichen Triglyceriden; synthetischen Triglyceriden; und Mischungen davon.

3. Körperpflegezusammensetzung nach einem der vorstehenden Ansprüche, wobei n zwischen 1,1 und 2,5 liegt, oder vorzugsweise, wobei n 2 ist.

4. Körperpflegezusammensetzung nach einem der vorstehenden Ansprüche, wobei die Nutzphase frei von Tensid ist.

5. Körperpflegezusammensetzung nach einem der vorstehenden Ansprüche, wobei die strukturierte Reinigungsphase ein zwitterionisches Tensid umfasst.

6. Körperpflegezusammensetzung nach einem der vorstehenden Ansprüche, wobei das strukturierende System ferner einen Elektrolyt umfasst.

7. Körperpflegezusammensetzung nach Anspruch 6, wobei der Elektrolyt ein Anion umfasst, ausgewählt aus der Gruppe bestehend aus Phosphat, Chlorid, Sulfat, Citrat und Mischungen davon; und ein Kation, ausgewählt aus der Gruppe bestehend aus Natrium, Ammonium, Kalium, Magnesium und Mischungen davon.

8. Körperpflegezusammensetzung nach Anspruch 6, wobei der Elektrolyt ausgewählt ist aus der Gruppe bestehend aus Natriumchlorid, Ammoniumchlorid, Natriumsulfat, Ammoniumsulfat und Mischungen davon.

9. Körperpflegezusammensetzung nach einem der vorstehenden Ansprüche, wobei das nichtassoziative Polymer ausgewählt ist aus der Gruppe bestehend aus Polysacchariden, synthetischen Kohlenwasserstoffpolymeren und Kombinationen davon.

10. Körperpflegezusammensetzung nach Anspruch 9, wobei das Polysaccharid Xanthangummi umfasst.

11. Körperpflegezusammensetzung nach einem der vorstehenden Ansprüche, wobei die strukturierte Reinigungsphase von 7 bis 18 Gew.-%, vorzugsweise von 9 bis 16 Gew.-% oder mehr bevorzugt von 11 bis 14 Gew.-%, bezogen auf das Gewicht der Körperpflegezusammensetzung, Natriumtrideceth-(n)-Sulfat umfasst, wobei n zwischen 0,5 und 2,7 liegt.

12. Körperpflegezusammensetzung nach einem der vorstehenden Ansprüche, wobei das strukturierende System von 0,05 bis 1 Gew.-%, vorzugsweise von 0,07 bis 0,5 Gew.-% oder mehr bevorzugt von 0,1 bis 0,3 Gew.-%, bezogen auf das Gewicht der Körperpflegezusammensetzung, das nichtassoziative Polymer umfasst.

**Revendications**

1. Composition de soins personnels comprenant :

a) une phase nettoyante structurée comprenant :

i) de 5 % à 20 %, en poids de la composition de soins personnels, de tridéceth (n) sulfate de sodium où n est compris entre 0,5 et 2,7 ;
ii) un agent tensioactif amphotère, un agent tensioactif zwittérionique, ou une combinaison de ceux-ci ; et
iii) un système structurant comprenant de 0,01 % à 0,03 %, en poids de la composition de soins personnels, d'un polymère associatif et de 0,01 % à 5 %, en poids de la composition de soins personnels, d'un polymère non associatif, dans laquelle le polymère associatif comprend des polymères réticulés acrylate/acrylate d'alkyle en C10 à C30 ; et

b) une phase bénéfique comprenant de 0,1 % à 50 %, en poids de la composition de soins personnels, d'un agent bénéfique ;

dans laquelle la phase nettoyante structurée et la phase bénéfique sont en contact physique.

2. Composition de soins personnels selon la revendication 1, dans laquelle l'agent bénéfique est choisi dans le groupe constitué de polyesters de saccharose ; vaseline ; huile minérale ; huile de soja ; lanoline ; dérivés de lanoline ; esters de lanoline ; huile de lanoline ; cires naturelles ; cires synthétiques ; organosiloxanes volatils ; dérivés d'organosiloxanes volatils ; organosiloxanes non volatils ; dérivés d'organosiloxanes non volatils ; triglycérides naturels ; triglycérides synthétiques ; et des combinaisons de ceux-ci.

3. Composition de soins personnels selon une quelconque revendication précédente, dans laquelle n est compris entre 1,1 et 2,5, ou de préférence où n vaut 2.

4. Composition de soins personnels selon une quelconque revendication précédente, dans laquelle la phase bénéfique est exempte d'agent tensioactif.

5. Composition de soins personnels selon une quelconque revendication précédente, dans laquelle la phase nettoyante structurée comprend un agent tensioactif zwittérionique.

6. Composition de soins personnels selon une quelconque revendication précédente, dans laquelle le système structurant comprend en outre un électrolyte.

7. Composition de soins personnels selon la revendication 6, dans laquelle l'électrolyte comprend un anion choisi dans le groupe constitué de phosphate, chlorure, sulfate, citrate et des mélanges de ceux-ci ; et un cation choisi dans le groupe constitué de sodium, ammonium, potassium, magnésium et des mélanges de ceux-ci.

8. Composition de soins personnels selon la revendication 6, dans laquelle l'électrolyte est choisi dans le groupe constitué de chlorure de sodium, chlorure d'ammonium, sulfate de sodium, sulfate d'ammonium, et des mélanges de ceux-ci.

9. Composition de soins personnels selon une quelconque revendication précédente, dans laquelle le polymère non associatif est choisi dans le groupe constitué de polysaccharides, polymères d'hydrocarbure synthétiques, et des combinaisons de ceux-ci.

10. Composition de soins personnels selon la revendication 9, dans laquelle le polysaccharide comprend de la gomme de xanthane.

11. Composition de soins personnels selon une quelconque revendication précédente, dans laquelle la phase nettoyante structurée comprend de 7 % à 18 %, de préférence de 9 % à 16 %, ou plus préférablement de 11 % à 14 %, en poids de la composition de soins personnels, de tridéceth (n) sulfate de sodium où n est compris entre 0,5 et 2,7.

12. Composition de soins personnels selon une quelconque revendication précédente, dans laquelle le système structurant comprend de 0,05 % à 1 %, de préférence de 0,07 % à 0,5 %, ou plus préférablement de 0,1 % à 0,3 %, en poids de la composition de soins personnels, du polymère non associatif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060079417 A
- US 20040223991 A1
- US 157665 A
- US 6335312 B, Coffindaffer
- US 7119059 B, Librizzi
- US 6897253 B, Schmucker-Castner
- US 817786 A
- US 5104646 A
- US 5106609 A
- US 2658072 A
- US 2438091 A
- US 2528378 A

- US 3929678 A
- US 5011681 A
- US 20040092415 A1
- US 5487884 A, Bisset
- US 5681852 A
- US 5652228 A, Bisset
- US 6395691 B, Liang Sheng Tsaur
- US 6645511 B, Aronson
- US 6759376 B, Zhang
- US 6780826 B, Zhang
- US 20060182699 A
- US 510880 A

### Non-patent literature cited in the description

- **MCCUTCHEON'S.** Detergents and Emulsifiers North American Edition. Allured Publishing Corporation 1947, 1986
- **MCCUTCHEON'S.** Functional Materials North American Edition. Allured Publishing Corporation 1973, 1992

- CTFA Cosmetic Ingredient Handbook. The Cosmetic, Toiletries, and Fragrance Association, Inc, 1988